# FASCICULE DE BREVET EUROPEEN

(11) **EP 1 405 654 B1**
(45) Date de publication et mention de la délivrance du brevet: **26.04.2006**
(21) Numéro de dépôt: 03292429.2
(22) Date de dépôt: 02.10.2003
(51) Int. Cl.: A61N 1/365

(54) **Dispositif médical implantable actif tel que stimulateur cardiaque, défibrillateur, cardioverteur et/ou dispositif multisite, comprenant des moyens de détermination d'un indice hémodynamique moyen**
Aktives implantierbares medizinisches Gerät, wie Herzschrittmacher, Defibrillatoren, Kardiovertierer und/oder Multisitevorrichtungen, mit Mitteln zur Feststellung der durchschnittlichen hemodynamischen Kennzahl
Active implantable medical devices, such as defibrillators, cardioverters and/or multisite devices, comprising means for determining the average hemodynamic index

(30) Priorité: 04.10.2002 FR 0212290
(43) Date de publication de la demande: 07.04.2004
(73) Titulaire: ELA MEDICAL, 92541 Montrouge (FR)
(72) Inventeur: Casset, Cyril, 75010 Paris (FR)
(74) Mandataire: Dupuis-Latour, Dominique

(56) Documents cités:
- EP-A- 1 116 497
- EP-A- 1 138 346

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle peut avantageusement concerner, plus particulièrement, les prothèses dites "multisite" dans lesquelles des électrodes sont placées en une pluralité de sites respectifs distincts comportant au moins un site ventriculaire et un site auriculaire. Il peut s'agir d'une prothèse de type "double chambre" (stimulation atriale droite et stimulation ventriculaire droite) ou, le plus souvent, "triple chambre" (stimulation atriale droite et double stimulation ventriculaire) ou "quadruple chambre" (double stimulation atriale et double stimulation ventriculaire).

Le pilotage de la stimulation implique l'ajustement permanent de divers paramètres tels que fréquence de stimulation, délai atrioventriculaire (DAV), ou encore délai interventriculaire dans le cas d'une stimulation biventriculaire. Ces divers paramètres sont ajustés en fonction de signaux délivrés par des capteurs, par exemple de la ventilation-minute (MV), qui est un facteur représentatif des besoins métaboliques instantanés du patient.

Un autre facteur qu'il peut être souhaitable de connaître est le débit cardiaque car il est possible, tout particulièrement avec un stimulateur multisite, d'obtenir une indication de ce débit et donc de la fraction d'éjection, qui est le paramètre hémodynamique de référence pour optimiser la stimulation sur les différents sites.

Le EP-A-1 116 497 (ELA Médical) décrit une manière d'effectuer une mesure dynamique de bioimpédance permettant d'évaluer les volumes diastolique et systolique, et d'obtenir ainsi une indication du débit cardiaque et donc de la fraction d'éjection. Le signal obtenu peut être utilisé pour piloter la fréquence cardiaque et/ou le délai atrioventriculaire dans le sens de la maximisation du débit. II est également possible d'utiliser le paramètre ainsi mesuré pour piloter le délai interventriculaire, dans le cas d'une stimulation biventriculaire.

Ce document décrit plus précisément une technique de mesure de la bioimpédance transvalvulaire (entre oreillette et ventricule situés d'un même côté du coeur) par une configuration tripolaire, avec injection d'une impulsion de courant entre un site atrial et un site ventriculaire, et recueil d'un potentiel différentiel entre un site atrial et un site ventriculaire, avec l'un des sites commun à l'injection et au recueil, un site propre d'injection et un site propre de recueil. Le courant injecté est un courant de faible amplitude, insuffisant pour exciter les cellules cardiaques.

Le EP-A-1 138 346 (ELA Médical) décrit un autre type de mesure de bioimpédance, qui est une bioimpédance transseptale, c'est-à-dire entre un site situé d'un côté du coeur et un site situé de l'autre côté du coeur. Cette technique permet encore de délivrer une valeur représentative de la fraction d'éjection, bien que le signal soit plus faible que dans le cas de la mesure d'une bioimpédance transvalvulaire, et soit également influencé par l'impédance propre des tissus du septum.

L'un des buts de la présente invention est d'utiliser cette mesure d'impédance intracardiaque pour déterminer, dans des conditions standardisées préétablies, un indice représentatif de l'évolution du débit cardiaque du patient sur le long terme.

Cet indice (ci-après "indice hémodynamique moyen"), déterminé à intervalles réguliers par exemple quotidiennement, pourra être mémorisé dans le dispositif pour utilisation ultérieure par un praticien à des fins diagnostiques.

Il est en effet apparu aux inventeurs que, sous des conditions strictes de temps, de rythme cardiaque et d'état du capteur d'asservissement dont peut être pourvu le dispositif, les variations quotidiennes de l'indice hémodynamique moyen qui seront obtenues par la mesure de bioimpédance intracardiaque sont représentatives de l'évolution de l'état du coeur droit du patient. Ces variations reflètent également, de manière indirecte, ce qui se passe sur les poumons, le coeur gauche et les tissus oxygénés du patient, du fait du retentissement général sur l'organisme du débit sanguin dans le coeur droit.

Ainsi, dans le cas d'un stimulateur double chambre, si l'évolution de l'indice moyen révèle une détérioration de l'état hémodynamique du patient, mais que l'activité cardiaque reste satisfaisante, le praticien pourra soupçonner une insuffisance pulmonaire.

L'invention permet également d'établir un diagnostic plus précoce de l'insuffisance cardiaque et/ou d'effectuer des comparaisons de l'état hémodynamique du patient dans des conditions récurrentes et bien déterminées avec modification du mode de fonctionnement du dispositif : on peut ainsi envisager, si le patient ne présente pas de trouble cardiaque important, de réaliser des mesures de bioimpédance quotidiennes pendant une semaine en mode stimulé, puis pendant une semaine en mode non stimulé (en laissant s'exprimer le rythme naturel spontané), pour comparer ensuite les deux séries de mesure afin d'évaluer la pertinence et l'efficacité d'une stimulation permanente.

A cet effet, la présente invention propose un dispositif du type général connu d'après le EP-A-1 116 497 précité, comprenant au moins un site ventriculaire et un site auriculaire, et des moyens pour délivrer un signal d'impédance intracardiaque corrélé au débit sanguin instantané, ces moyens opérant par injection d'un courant et recueil d'un potentiel différentiel à des bornes respectives d'une configuration desdits sites.

Selon l'invention, ce dispositif est caractérisé par des moyens pour déterminer périodiquement, à partir du signal d'impédance, un indice hémodynamique moyen évalué sur plusieurs cycles dans des conditions de mesure prédéfinies, ces moyens comprenant : des moyens de contrôle, pour vérifier que l'état du patient et du dispositif répond à une batterie de critères définissant des conditions de mesure prédéterminées, et inhiber la détermination dudit indice si ces critères ne sont pas remplis ;des moyens de mesure, pour recueillir une pluralité d'échantillons du signal d'impédance pendant la durée de la systole d'un cycle cardiaque ; des moyens intégrateurs, pour déterminer, à partir desdits échantillons, une grandeur représentative du volume sanguin éjecté pendant la durée de cette systole ; et des moyens de calcul, pour établir ledit indice hémodynamique moyen à partir d'une pluralité desdites grandeurs successivement déterminées par les moyens intégrateurs sur une pluralité de cycles cardiaques.

Avantageusement, les moyens de mesure comprennent des moyens pour déterminer l'instant de début d'une systole par détection d'un événement cardiaque ventriculaire prédéterminé, et pour déterminer la fin de cette systole lorsque le signal d'impédance atteint un maximum au cours du même cycle cardiaque, les moyens intégrateurs pouvant en particulier comprendre des moyens pour déterminer la grandeur représentative du volume sanguin éjecté par évaluation de l'augmentation de l'aire du signal d'impédance entre l'instant de début et l'instant de fin de la systole.

Les moyens de calcul peuvent notamment comprendre de moyens pour déterminer l'indice hémodynamique par moyennage des valeurs successives des grandeurs représentatives pondérées par la durée des systoles associées à ces grandeurs.

De préférence, les moyens pour délivrer le signal d'impédance intracardiaque sont des moyens pour délivrer un signal d'impédance transvalvulaire, ces moyens opérant par injection d'un courant entre un site atrial et un site ventriculaire situés du même côté du coeur et recueil d'un potentiel différentiel entre un site atrial et un site ventriculaire également situés de ce même côté du coeur.

Les moyens de contrôle peuvent notamment comprendre des moyens pour vérifier :
- que l'heure courante est située à l'intérieur d'une fenêtre temporelle prédéterminée ;
- que l'événement ventriculaire et l'événement auriculaire associés au cycle cardiaque courant forment un couple d'événements d'un type attendu prédéterminé ;
- que la fréquence cardiaque associée au cycle courant est comprise entre deux bornes prédéterminées ;
- qu'un capteur d'asservissement du dispositif indique un état prédéterminé du patient ; et/ou
- que se sont succédés un nombre prédéterminé de cycles cardiaques antérieurs pour lesquels l'état du patient et du dispositif ont répondu à la batterie de critères.

On va maintenant décrire un exemple de mise en oeuvre de l'invention, en référence aux dessins annexés.
La figure 1 est un chronogramme donnant l'évolution, au cours de deux cycles cardiaques successifs, de la pression cardiaque intraventriculaire et de la bioimpédance transvalvulaire.
La figure 2 est un chronogramme montrant la manière dont évolue la bioimpédance au cours d'un cycle cardiaque, et dont on opère les divers échantillonnages et mesures nécessaires à la mise en oeuvre de l'invention.
La figure 3 est un organigramme explicitant les différentes étapes de la mise en oeuvre de l'invention.

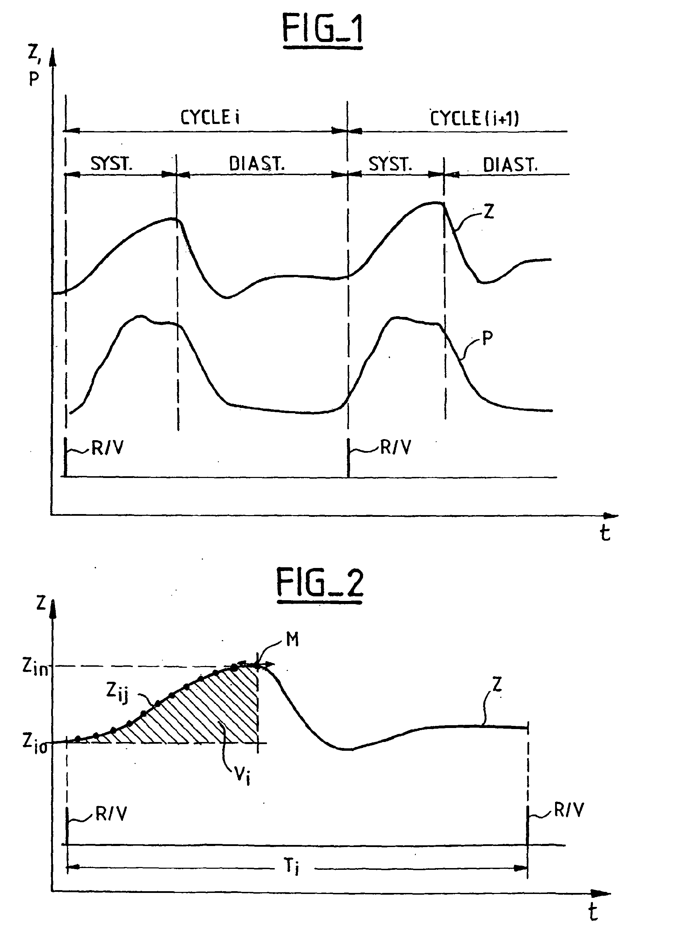

Sur la figure 1, la caractéristique référencée P illustre l'évolution dans le temps de la pression cardiaque dans le ventricule droit, avec la succession des phases systolique et diastolique du cycle cardiaque.

Pendant la systole, la pression augmente rapidement puis se stabilise aux alentours de sa valeur maximale, ce plateau de la caractéristique correspondant au début de l'ouverture des valvules. Les cavités se vident ensuite pendant la durée de la diastole, jusqu'à l'événement ventriculaire suivant, spontané (événement R) ou stimulé (événement V). La durée d'un cycle cardiaque sera définie comme l'intervalle de temps séparant deux événements ventriculaires successifs spontanés ou stimulés.

On a porté sur ce même chronogramme les variations de l'impédance intracardiaque Z, dans cet exemple l'impédance transvalvulaire (choisie de préférence à une impédance transseptale, pour les raisons indiquées plus haut), et l'on voit que les variations de cette impédance transvalvulaire Z sont étroitement corrélées aux variations de la pression cardiaque. En particulier, la caractéristique d'impédance transvalvulaire présente un maximum correspondant précisément à la fin de la systole ventriculaire, que l'on pourra ainsi aisément détecté par ce biais (la fin de la systole ventriculaire correspond à l'état de contraction maximale, donc à la situation où le volume sanguin est le plus faible, avec par voie de conséquence une valeur d'impédance transvalvulaire élevée).

En référence à la figure 2, qui illustre la variation de l'impédance transvalvulaire Z au cours d'un cycle cardiaque de durée Tᵢ (durée définie, comme indiqué plus haut, comme étant l'intervalle de temps séparant deux événements ventriculaires R ou V), l'invention propose d'évaluer le volume éjecté au cours de la phase systolique de ce i^{ième} cycle à partir de la variation de l'impédance Z.

Ce volume est proportionnel (à un facteur constant près, dont on fera abstraction) à l'aire Vᵢ hachurée sur la figure 2, c'est-à-dire à l'accroissement de l'aire située sous la courbe d'impédance Z, cet accroissement étant compté entre, d'une part, le début du cycle cardiaque (lorsque le dispositif détecte la survenue de l'événement ventriculaire R ou V) et, d'autre part, la fin de la systole, détectée par le changement du sens de variation de la courbe d'impédance Z (maximum M).

Cette aire Vᵢ pourra être évaluée par intégration du signal Z à partir d'échantillons successifs Zᵢⱼ recueillis entre l'instant de début du cycle cardiaque (échantillon Zᵢ₀) et le point où la courbe d'impédance atteint son maximum (échantillon Zᵢₙ).

Le volume Vᵢ et la durée Tᵢ du cycle associé permettront ainsi de calculer, de la manière que l'on indiquera plus bas, un indice hémodynamique Dᵢ associé à ce i^{ième} cycle. Ce processus, répété sur un nombre prédéterminé de cycles, permettra d'obtenir un indice hémodynamique moyen D_{moy} qui sera ensuite mémorisé dans l'appareil pour utilisation ultérieure par le praticien ou par un logiciel d'analyse approprié.

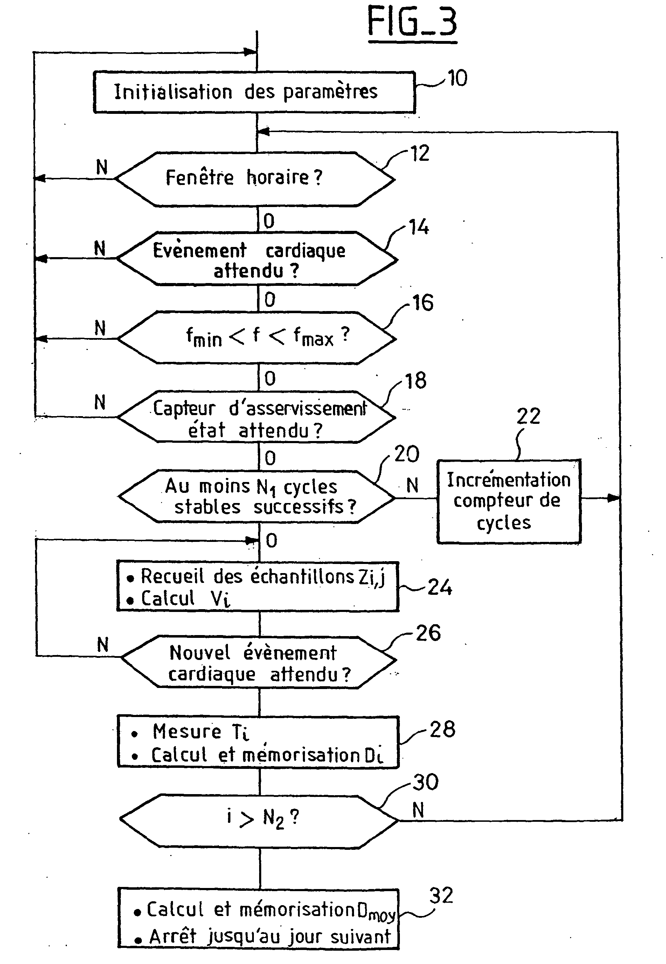

L'organigramme de la figure 3 illustre les diverses étapes de la mise en oeuvre de l'invention.

Comme on l'a indiqué, on cherche à déterminer un indice hémodynamique sous des conditions récurrentes et bien déterminées, et ceci à intervalles réguliers (par exemple quotidiennement), de manière à pouvoir en évaluer ensuite l'évolution sur le long terme.

II est donc nécessaire, avant toute mesure, de vérifier que ces conditions particulières sont bien toutes remplies, et maintenues tout au long de la mesure.

Après initialisation des divers paramètres de l'algorithme (étape 10), celui-ci détermine tout d'abord si l'heure courante correspond ou non à la fenêtre horaire prédéterminée au cours de laquelle la mesure doit être effectuée (étape 12). On peut par exemple programmer le dispositif pour que cette mesure soit réalisée dans une phase où l'on est à peu près certain que le patient sera au repos et dans un état physiologique comparable d'un jour à l'autre, en définissant par exemple une fenêtre horaire comprise entre minuit et trois heures du matin.

Pour une description de moyens permettant de déterminer ces phases de repos, on pourra par exemple se référer au EP-A-0 719 568 (ELA Medical), qui décrit la détection de phases de repos à partir du signal issu d'un capteur physiologique tel qu'un capteur de ventilation-minute, ou au EP-A-0 750 920 (ELA Medical), où le capteur physiologique est combiné à un capteur d'activité tel qu'un accéléromètre pour accroître la spécificité de la détection, ou encore au EP-A-1 317 943 (ELA Medical) qui décrit la détection de phases de repos en vue de déterminer un état de sommeil du patient.

Si ce premier critère est vérifié, l'algorithme examine ensuite si, lorsqu'un événement ventriculaire est détecté, celui-ci correspond bien au type d'événement attendu (étape 14).

En effet, dans la mesure où la stimulation modifie l'hémodynamique du patient, le rythme doit être de la même nature pour tous les cycles sur lesquels est opérée la mesure. Ce paramètre peut être choisi parmi quatre rythmes possibles : P-R, P-V, A-R ou A-V (P et A désignant des événements auriculaires respectivement spontanés et stimulés, et R et V désignant des événements ventriculaires respectivement spontanés et stimulés).

L'algorithme examine ensuite si la fréquence f du rythme cardiaque est comprise entre deux bornes fₘᵢₙ et fₘₐₓ prédéterminées entre lesquelles le cycle courant peut être considéré comme exploitable aux fins du calcul de l'indice hémodynamique (étape 16). Par exemple, on définit que la fréquence f doit être comprise entre 60 et 65 cpm, ou entre 60 et 70 cpm, ou tout autre plage de valeurs relativement étroite, fonction de la physiologie propre du patient.

L'étape suivante (étape 18) consiste à vérifier que, dans le cas où le dispositif est pourvu d'un ou plusieurs capteurs d'asservissement, celui-ci se trouve dans un état prédéterminé, par exemple un état définissant une phase de repos.

L'algorithme vérfie ensuite (étape 20) que les divers critères que l'on vient d'exposer ont tous été cumulativement vérifiés au cours d'un nombre N₁ de cycles cardiaques successifs, par exemple N₁ = 10 cycles successifs. Dans le cas contraire, un compteur de cycles est incrémenté (étape 22) et les tests des étapes 12 à 18 sont réitérés.

Sinon, c'est-à-dire si l'on a vérifié les critères de stabilité sur au moins N₁ cycles successifs, le dispositif est prêt à commencer l'enregistrement de l'impédance et le traitement des valeurs qui vont être enregistrées.

La première étape consiste à recueillir les échantillons d'impédance Zᵢⱼ et à intégrer la valeur, variable, de la courbe d'impédance de manière à déterminer l'aire Vᵢ définie plus haut en référence à la figure 2 (étape 24), jusqu'à détection du maximum M.

L'algorithme attend alors l'événement cardiaque suivant, en vérifiant (étape 26) que celui-ci correspond bien au type de rythme prédéfini (P-R, P-V, A-R ou A-V).

Dans la négative, la mesure n'est pas conservée, elle est recommencée au cycle suivant.

Dans l'affirmative, l'algorithme détermine la durée Tᵢ du cycle cardiaque qui vient de se terminer, et en déduit l'indice hémodynamique Dᵢ correspondant à ce cycle (étape 28). Cet indice Dᵢ est déterminé comme étant le quotient Dᵢ=Vᵢ/Tᵢ du volume éjecté Vᵢ par la durée du cycle Tᵢ ; il est en effet nécessaire, pour connaître l'état hémodynamique réel du patient, de pondérer le volume éjecté au cours d'un cycle par la durée de ce cycle, l'état hémodynamique le meilleur correspondant à un volume important éjecté lors d'un cycle de courte durée.

L'indice hémodynamique Dᵢ est ainsi déterminé sur une pluralité d'un nombre N₂ de cycles successifs, par exemple sur N₂ = 10 cycles successifs (étape 30).

Une fois cette série de mesures achevée, l'algorithme calcule alors (étape 32) la valeur de l'indice hémodynamique moyen D_{moy}, qui est la moyenne arithmétique des indices Dᵢ des N₂ cycles considérés. Cette indice hémodynamique moyen journalier est alors mémorisé, ce qui met fin au processus jusqu'au jour suivant.

## Revendications

1. Un dispositif médical implantable actif, notamment un stimulateur cardiaque, défibrillateur et/ou cardioverteur et/ou dispositif multisite dans lequel des électrodes sont placées en une pluralité de sites respectifs distincts comportant au moins un site ventriculaire et un site auriculaire,
ce dispositif comportant des moyens pour délivrer un signal d'impédance intracardiaque corrélé au débit sanguin instantané, ces moyens opérant par injection d'un courant et recueil d'un potentiel différentiel à des bornes respectives d'une configuration desdits sites,
dispositif **caractérisé par** des moyens pour déterminer périodiquement, à partir du signal d'impédance (Z), un indice hémodynamique moyen (D_{moy}) évalué sur plusieurs cycles dans des conditions de mesure prédéfinies, ces moyens comprenant :
- des moyens de contrôle pour vérifier que l'état du patient et du dispositif répond à une pluralité de critères (12-20) définissant des conditions de mesure prédéterminées, et inhiber la détermination dudit indice si ces critères ne sont pas remplis ;
- des moyens de mesure, pour recueillir une pluralité d'échantillons (Zᵢⱼ) du signal d'impédance pendant la durée (Tᵢ) de la systole (SYST.) d'un cycle cardiaque ;
- des moyens intégrateurs, pour déterminer, à partir desdits échantillons, une grandeur (Dᵢ) représentative du volume sanguin éjecté pendant la durée de cette systole ; et
- des moyens de calcul, pour établir ledit indice hémodynamique moyen (D_{moy}) à partir d'une pluralité desdites grandeurs successivement déterminées par les moyens intégrateurs sur une pluralité (N₂) de cycles cardiaques.

2. Le dispositif de la revendication 1, dans lequel les moyens de mesure comprennent des moyens pour déterminer l'instant de début d'une systole par détection d'un événement cardiaque ventriculaire prédéterminé (R, V), et pour déterminer la fin de cette systole lorsque le signal d'impédance atteint un maximum (Zᵢₙ) au cours du même cycle cardiaque.

3. Le dispositif de la revendication 2, dans lequel les moyens intégrateurs comprennent des moyens pour déterminer ladite grandeur représentative du volume sanguin éjecté par évaluation de l'augmentation de l'aire (Vᵢ) du signal d'impédance entre l'instant de début et l'instant de fin de la systole.

4. Le dispositif de la revendication 1, dans lequel les moyens de calcul comprennent de moyens pour déterminer ledit indice hémodynamique par moyennage des valeurs successives des grandeurs représentatives (Dᵢ) pondérées par la durée (Tᵢ) des systoles associées à ces grandeurs.

5. Le dispositif de la revendication 1, dans lequel les moyens pour délivrer le signal d'impédance intracardiaque sont des moyens pour délivrer un signal d'impédance transvalvulaire, ces moyens opérant par injection d'un courant entre un site atrial et un site ventriculaire situés du même côté du coeur et recueil d'un potentiel différentiel entre un site atrial et un site ventriculaire également situés de ce même côté du coeur.

6. Le dispositif de la revendication 1, dans lequel les moyens de contrôle comprennent des moyens pour vérifier (12) que l'heure courante est située à l'intérieur d'une fenêtre temporelle prédéterminée.

7. Le dispositif de la revendication 1, dans lequel les moyens de contrôle comprennent des moyens pour vérifier (14, 26) que l'événement ventriculaire et l'événement auriculaire associés au cycle cardiaque courant forment un couple d'événements d'un type attendu prédéterminé.

8. Le dispositif de la revendication 1, dans lequel les moyens de contrôle comprennent des moyens pour vérifier (16) que la fréquence cardiaque (f) associée au cycle courant est comprise entre deux bornes (fₘᵢₙ, fₘₐₓ) prédéterminées.

9. Le dispositif de la revendication 1, dans lequel les moyens de contrôle comprennent des moyens pour vérifier (18) qu'un capteur d'asservissement du dispositif indique un état prédéterminé du patient.

10. Le dispositif de la revendication 1, dans lequel les moyens de contrôle comprennent des moyens pour vérifier (20) que se sont succédés un nombre (N₁) prédéterminé de cycles cardiaques antérieurs pour lesquels l'état du patient et du dispositif ont répondu à ladite batterie de critères.

## Patentansprüche

1. Aktive implantierbare medizinische Vorrichtung, insbesondere Herzschrittmacher, Defibrillator und/oder Kardioverter und/oder "Multisite"-Vorrichtung, in welcher Elektroden an einer Mehrzahl von jeweiligen unterschiedlichen Sitzen angeordnet sind, die wenigstens einen Herzkammersitz und einen Herzvorhofsitz umfassen,
wobei diese Vorrichtung Mittel umfasst, um ein Signal intrakardialer Impedanz abzugeben, das mit dem augenblicklichen Blutdurchsatz korreliert,
wobei diese Mittel durch Einleitung eines Stromflusses und Erfassung eines Spannungsunterschiedes an jeweiligen Anschlüssen einer Anordnung der Sitze arbeiten,
wobei die Vorrichtung durch Mittel zur periodischen Bestimmung, auf der Grundlage des Impedanzsignals (Z) einer durchschnittlichen hämodynamisehen Kennzahl (Dₘᵢₜₜₑₗ), welche unter vorbestimmten Messbedingungen über mehrere Zyklen ermittelt wird, **gekennzeichnet** ist, wobei diese Mittel Folgendes umfassen:
- Kontrollmittel, um nachzuprüfen, dass der Zustand des Patienten und der Vorrichtung einer Mehrzahl von Kriterien (12-20) entspricht, die vorbestimmte Messbedingungen definieren, und um die Bestimmung der Kennzahl zu hemmen, wenn diese Kriterien nicht erfüllt sind;
- Messmittel, um eine Mehrzahl von Proben (Zᵢⱼ) des Impedanzsignals während der Dauer (Tᵢ) der Systole (SYST.) eines Herzzyklusses aufzunehmcn;
- Integriermittel, um anhand der Proben eine Größe (Dᵢ) festzustellen, die für das während der Dauer dieser Systole ausgeworfene Blutvolumen repräsentativ ist; und
- Berechnungsmittel, um die durchschnittliche hämodynamische Kennzahl (Dₘᵢₜₜₑₗ) anhand einer Mehrzahl der Größen zu ermitteln, die sukzessiv durch die Integrielmittel über eine Mehrzahl (N₂) von Herzzyklen bestimmt werden.

2. Vorrichtung nach Anspruch 1, in welcher die Messmittel Mittel umfassen, um den Augenblick des Beginns einer Systole durch Erfassung eines vorbestimmten Herzkammerereignisses (R, V) zu bestimmen, und um das Ende dieser Systole zu bestimmen, wenn das Impedanzsignal während desselben Herzzyklusses ein Maximum (Zᵢₙ) erreicht.

3. Vorrichtung nach Anspruch 2, in welcher die Integriermittel Mittel umfassen, um die repräsentative Größe des ausgeworfenen Blutvolumens durch Ermittlung der Vergrößerung der Fläche (Vᵢ) des Impedanzsignals zwischen dem Augenblick des Beginns und dem Augenblick des Endes der Systole zu bestimmen.

4. Vorrichtung nach Anspruch 1, in welcher die Bercchnungsmittel Mittel umfassen, um die hämodynamische Kennzahl durch Mittlung der sukzessiven Werte der repräsentativen Größen (Dᵢ), gewichtet durch die Dauer (Tᵢ) der diesen Größen zugeordneten Systolen, zu bestimmen.

5. Vorrichtung nach Anspruch 1, in welcher die Mittel zur Abgabe des intrakardialen Impedanzsignals Mittel sind, um ein Zwischenklappen-Impedanzsignal abzugeben, wobei diese Mittel durch Einleitung eines Stromflusses zwischen einem Vorhofsitz und einem Kammersitz, die sich auf derselben Seite des Herzens befinden, und Erfassung eines Spannungsunterschieds zwischen einem Herzvorhofsitz und einem Kammersitz, die sich ebenfalls auf dieser selben Seite des Herzens befinden, arbeiten.

6. Vorrichtung nach Anspruch 1, in welcher die Kontrollmittel Mittel umfassen, um nachzuprüfen (12), dass die gegenwärtige Uhrzeit sich innerhalb eines vorbestimmten Zeitfensters befindet.

7. Vorrichtung nach Anspruch 1, in welcher die Kontrollmittel Mittel umfassen, um nachzuprüfen (14, 26), dass das Kammerereignis und das Vorhofereignis, welche dem gegenwärtigen Herzzyklus zugeordnet sind, ein Ereignispaar eines erwarteten vorbestimmten Typs bilden.

8. Vorrichtung nach Anspruch 1, in welcher die Kontrollmittel Mittel umfassen, um nachzuprüfen (16), dass die dem gegenwärtigen Zyklus zugeordnete Herzfrequenz (f) zwischen zwei vorbestimmten Grenzwerten (fₘᵢₙ, fₘₐₓ) liegt.

9. Vorrichtung nach Anspruch 1, in welcher die Kontrollmittel Mittel umfassen, um nachzuprüfen (18), dass ein Steuerungssensor der Vorrichtung einen vorbestimmten Zustand des Patienten anzeigt.

10. Vorrichtung nach Anspruch 1, in welcher die Kontrollmittel Mittel umfassen, um nachzuprüfen (20), dass eine vorbestimmte Anzahl (N₁) vorhergehender Herzzyklen aufeinander gefolgt sind, bei welchen der Zustand des Patienten und der Vorrichtung der Reihe an vorgenannten Kriterien entsprach.

## Claims

1. An active implantable medical device, in particular a cardiac pacemaker, defibrillator, and/or cardioverter and/or multisite device, wherein electrodes are placed in a plurality of distinct respective sites including at least one ventricular site and one atrial site,
said device comprising means for delivering an intracardiac impedance signal correlated to the instantaneous blood output, wherein said means operates by injecting a current and collecting a differential potential at respective terminals of a configuration of said sites,
said device being **characterised by** means for determining periodically, on the basis of said impedance signal (Z), an average hemodynamic index (D_{average}) evaluated over several cycles under predefined measurement conditions, said means including:
- control means for checking that the state of the patient and of the device corresponds to a plurality of conditions (12-20) defining predetermined measurement conditions and for inhibiting the determination of said index if said criteria are not satisfied;
- measuring means for collecting a plurality of samples (Zᵢⱼ) of the impedance signal over the duration (Tᵢ) of the systole (SYST.) of one cardiac cycle;
- integrating means for determining, on the basis of said samples, a quantity (Dᵢ) representative of the blood volume ejected throughout the duration of said systole; and
- calculating means for establishing said average hemodynamic index (D_{average}) on the basis of a plurality of said quantities successively determined by the integrating means over a plurality (N₂) of cardiac cycles.

2. The device of claim 1, wherein the measuring means comprises means for determining the moment at which a systole begins by detection of a predetermined ventricular cardiac event (R, V), and for determining the end of said systole when said impedance signal reaches a maximum (Zᵢₙ) during said same cardiac cycle.

3. The device of claim 2, wherein the integrating means comprises means for determining said quantity representative of the ejected blood volume by evaluation of the increase of the area (Vᵢ) of the impedance signal between said beginning and end of the systole.

4. The device of claim 1, wherein the calculating means comprises means for determining said hemodynamic index by averaging the successive values of said representative quantities (Dᵢ) weighted by the duration (Tᵢ) of the systoles associated to these quantities.

5. The device of claim 1, wherein the means for delivering the intracardiac impedance signal is a means for delivering a transvalvular impedance signal, said means operating by injecting a current between an atrial site and a ventricular site located on the same side of the heart, and collecting a differential potential between an atrial site and a ventricular site also located on said same side of the heart.

6. The device of claim 1, wherein said control means comprises means for verifying (12) that the current time is located inside a predetermined temporal window.

7. The device of claim 1, wherein said control means comprises means for verifying (14, 26) that the ventricular event and the atrial event associated with the current cardiac cycle form an event couple of a predetermined expected type.

8. The device of claim 1, wherein the control means comprises means for verifying (16) that the heart rate (f) associated with the current cycle is comprised between two predetermined limits (fₘᵢₙ, fₘₐₓ).

9. The device of claim 1, wherein the control means comprises means for verifying (18) that a control sensor of the device indicates a predetermined patient state.

10. The device of claim 1, wherein the control means comprises means for verifying (20) that a predetermined number (N₁) of preceding cardiac cycles have succeeded one another for which the state of the patient and of the device have satisfied said plurality of criteria.
